Europäisches Patentamt

European Patent Office

Office européen des brevets

(11) Veröffentlichungsnummer: **0 300 347 B1**

(12) **EUROPÄISCHE PATENTSCHRIFT**

(45) Veröffentlichungstag der Patentschrift: **08.07.92**

(51) Int. Cl.⁵: **C07C 29/136**, C07C 31/125

(21) Anmeldenummer: **88111205.6**

(22) Anmeldetag: **13.07.88**

(54) **Verfahren zur Hydrierung von Fettsäuremethylestergemischen.**

(30) Priorität: **22.07.87 DE 3724257**

(43) Veröffentlichungstag der Anmeldung:
**25.01.89 Patentblatt 89/04**

(45) Bekanntmachung des Hinweises auf die Patenterteilung:
**08.07.92 Patentblatt 92/28**

(84) Benannte Vertragsstaaten:
**AT BE CH DE ES FR GB GR IT LI LU NL SE**

(56) Entgegenhaltungen:
**EP-A- 0 254 189**
**FR-A- 1 276 722**
**US-A- 2 109 844**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Fleckenstein, Theo, Dr.**
**Pfitznerstrasse 9**
**W-4010 Hilden(DE)**
Erfinder: **Pohl, Joachim, Dr.**
**Leinenweberweg 23**
**W-4000 Düsseldorf(DE)**
Erfinder: **Carduck, Franz-Josef, Dr.**
**Landstrasse 18**
**W-5657 Haan(DE)**

**Beschreibung**

Die Erfindung betrifft ein neues Verfahren zur katalytischen Hydrierung von Fettsäuremethylestergemischen unter Verwendung stückiger und/oder gekörnter Kupferchromit enthaltender Katalysatoren im Druckbereich von 100 bis 300 bar.

Fettalkohole, d.h. überwiegend lineare, monofunktionelle Alkohole mit Kettenlägen von 8 und mehr C-Atomen, sowie ihre Herstellung sind in der Literatur ausführlich beschrieben, beispielsweise in Ullmanns Encyklopädie der technischen Chemie, 4. Auflage, Band 11, Seiten 427 bis 445. Ein bevorzugtes Ausgangsmaterial zu ihrer Gewinnung sind die in Fetten und/oder Ölen natürlichen Ursprungs vorkommenden Fettsäuren bzw. Fettsäuregemische, die durch katalytische Hydrierung in Fettalkohole entsprechender Kettenlänge umgewandelt werden können. Durch den Einsatz der zu reduzierenden Fettsäuren in Form ihrer Methylester werden insbesondere die Katalysatoren vor einem aggressiven Angriff durch die freie Carboxylgruppe geschützt, so daß in großtechnischen Verfahren für hinreichend lange Zeiträume mit befriedigenden Raumzeitausbeuten gearbeitet werden kann. Demgemäß wird die überwiegende Menge nativer Fettalkohole heute aus Fettsäuremethylestern nach dem Gasphasenhydrierverfahren hergestellt, bei dem die destillierten Methylester über fest angeordnete, kupferhaltige Mischoxid-Katalysatoren, wie beispielsweise Kupferchromit-Spinellkatalysatoren, in dampfförmigem Zustand zusammen mit einem großen Überschuß an Wasserstoff bei Temperaturen von oberhalb 200°C und Drucken von etwa 250 bis 300 bar geleitet werden.

Die auf feuchtem Wege durch gemeinsame Fällung hergestellten Kupfer-Mischoxid-Katalysatoren werden als stückige Kontakte oder Strangpreßlinge benutzt und vor Gebrauch meist in der Anlage reduziert.

In der einschlägigen Patentliteratur werden daher häufig Fettsäureester, insbesondere Fettsäuremethylester und freie Fettsäuren gleichzeitig als Einsatzmaterialien für die Hydrierungsreaktion zu gesättigten und/ oder ungesättigten Fettalkoholen verwendet. Verwiesen sei beispielsweise auf die DE-PSen 965 236, 10 05 497, 25 13 377 und 26 13 226. Für die großtechnische praktische Verwertung sind die genannten Vorschläge durchaus unterschiedlich zu bewerten, je nachdem, ob die Fettsäureester oder die freien Fettsäuren als Ausgangsmaterial der Hydrierung verwendet werden.

In der **FR-A 1 276 722** ist ein verfahren zur Herstellung eines Katalysators beschrieben, der für die Hydrierung von Estern von aliphatischen Carbonsäuren mit $C_1$-$C_6$-Monoalkoholen eingesetzt werden kann. Es wird vorgeschlagen, bekannte Katalysatormassen, die Kupferoxid, Chromoxid und gegebenenfalls Bariumoxid enthalten, unter Zuhilfenahme eines wasserlöslichen Bindemittels in stückige Form zu bringen, danach das Bindemittel vollständig auszulaugen, bevor der Katalysator in die Hydrierung eingesetzt wird. Angaben über die spezifische Oberfläche oder das Porenvolumen sind dieser Druckschrift nicht zu entnehmen; es wird jedoch davor gewarnt, bei der Verformung Graphit einzusetzen. Für die gemeinsame Hydrierung von $C_8$-$C_{22}$-Fettsäuremethylestern und die Lösung der damit verbundenen Probleme bietet das Dokument keine Anhaltspunkte.

Die Patentschrift **US 2,109,844** beschreibt die katalytische Direkthydrierung von Fettsäureglycerinestern zu den entsprechenden Alkoholen und enthält ebenfalls keinen Hinweis auf die gemeinsame Hydrierung von $C_8$-$C_{22}$-Fettsäuremethylestern oder darauf, wie ein solches Verfahren zu gestalten ist.

Nach dem gegenwärtigen Stand der Hydriertechnik muß nach erfolgter Umesterung der Öle und Fette und nach Abtrennung des Glycerins eine destillative Auftrennung der Methylester in einen Siedeschnitt im Kohlenstoffkettenbereich 8 bis 10 und in einen Siedeschnitt im Kohlenstoffkettenbereich 12 bis 18 vorgenommen werden, um bei der anschließenden Hydrierung der Methylester die zwangsläufig anfallenden Kohlenwasserstoffe entfernen zu können. Die beiden Fraktionen werden getrennt hydriert, wobei die qualitätsmindernden Kohlenwasserstoffe über Kopf abdestilliert werden. Da sich die Siedebereiche der Kohlenwasserstoffe mit denen der kurzkettigen Fettalkohole überschneiden, ist gemäß dem gegenwärtigen Stand der Technik eine Auftrennung der Fettsäuremethylester notwendig. Zudem ist die Reaktionsgeschwindigkeit bei der Hydrierung von kurzkettigen Fettsäuremethylestern geringer gegenüber langkettigen Fettsäuremethylestern. Demgemäß werden Fettsäuremethylester im Kohlenstoffkettenbereich 14 bis 16 bei einer gemeinsamen Hydrierung mit kürzerkettigen Fettsäuremethylestern überhydriert. Üblicherweise werden die aufgetrennten Kohlenstoffkettenbereiche mit unterschiedlichen Raumströmungsgeschwindigkeiten hydriert.

Es wurde nun überraschend gefunden, daß es mit bestimmten, Kupferchromit als Hauptbestandteil enthaltenden Katalysatoren mit hoher Aktivität und Selektivität unter langer Standzeit der Katalysatoren möglich ist, die Hydrierung von Fettsäuremethylestergemischen zu Fettalkoholen so zu steuern, daß die Bildung von Kohlenwasserstoffen weitgehend unterdrückt wird und gleichzeitig kürzerkettige und längerkettige Fettsäuremethylester hydriert werden können.

Aufgabe der vorliegenden Erfindung war es, ein Verfahren zur katalytischen Hydrierung von Fettsäuremethylestergemischen unter Verwendung stückiger und/oder gekörnter, Kupferchromit enthaltender Katalysatoren zur Verfügung zu stellen, mit dem Fettsäuremethylestergemische mit kurzkettigen und langkettigen Fettsäureresten im Kohlenstoffkettenbereich von 8 bis 22 Kohlenstoffatomen ohne vorherige Auftrennung der Fettsäuremethylestergemische in einzelne Siedeschnitte in hoher Ausbeute zu Fettalkoholen umgesetzt werden können. Der für die Umsetzung verwendete heterogene Übergangsmetall-Katalysator sollte mit hoher Aktivität und Selektivität zu den gewünschten Produkten führen, ohne daß Folgereaktionen in nennenswertem Maß zu einer Verminderung der Produktausbeute beitragen. Zusammen mit einer Einstellung schonender Reaktionsbedingungen sollte damit die Wirtschaftlichkeit des Verfahrens im Vergleich zum Stand der Technik verbessert werden.

Möglichst geringe Anteile von Kohlenwasserstoffen sind erwünscht, da bei der destillativen Aufarbeitung der Gemische der Fettalkohole Überschneidungen der Siedebereiche der Kohlenwasserstoffe mit denen der kurzkettigen Fettalkohole auftreten.

Die Erfindung betrifft ein Verfahren zur katalytischen Hydrierung von Fettsäuremethylestergemischen mit kurzkettigen und langkettigen Fettsäureresten im Kohlenstoffkettenbereich von 8 bis 22 Kohlenstoffatomen bei erhöhten Reaktionstemperaturen unter Verwendung stückiger und/oder gekörnter, Kupferchromit als Hauptbestandteil enthaltender Katalysatoren, das dadurch gekennzeichnet ist, daß man diese ohne vorherige Auftrennung in einzelne Siedeschnitte zusammen mit Wasserstoff bei Drucken von 100 bis 300 bar und Temperaturen von 160 bis 270°C bei Molverhaltnissen von Wasserstoff : Fettsäuremethylester-Einsatz von 10 : 1 bis 500 : 1 kontinuierlich über Katalysatoren umsetzt, die 30 bis 40 Gew.-% Kupfer, 23 bis 30 Gew.-% Chrom, 1 bis 10 Gew.-% Mangan und 1 bis 7 Gew.-% Barium sowie gegebenenfalls Zusätzlich 1 bis 5 Gew.-% Zirkon und/oder Cer - jeweils bezogen auf oxidische Katalysatormasse - enthalten und nach dem Calcinieren der die Katalysatormasse bildenden Komponenten mit 1 bis 10 Gew.-% - bezogen auf oxidischen Katalysator - wenigstens eines Binders und 1 bis 10 Gew.-% bezogen auf den oxidischen Graphit Katalysator - Graphit zu stückigen und/oder grobkörnigen Formlingen, die eine spezifische Oberfläche im Bereich von 30 bis 50 cm$^2$/g, ein Porenvolumen im Bereich von 0,4 bis 0,6 cm$^3$/g und bei stückigen Formlingen einen Durchmess von 1 bis 6 mm und eine Länge von 1 bis 6 mm, bei Gekörnten Formlingen eine Korngröße im Bereich von 0,6 bis 3,0 mm aufweisen, umgewandelt und mit Wasserstoff oder einem Wasserstoff enthaltenden Gasgemisch aktiviert worden sind.

Gemäß dem erfindungsgemäßen Verfahren katalytisch hydrierbare Fettsäuremethylestergemische können nativen oder synthetischen Ursprungs sein. Als Ausgangsstoffe für das erfindungsgemäße Verfahren zur Hydrierung kommen die aus tierischen oder pflanzlichen Quellen entstammenden Fette, Trane oder Öle in Frage, in denen einfach oder mehrfach ungesättigte Fettsäuren mit Glycerin verestert sind, wobei die Fettsäurereste gleiche oder unterschiedliche Sättigungsgrade bzw. Längen ihrer Alkylketten aufweisen können. Die Fettsäuremethylester können durch bekannte Verfahren aus den genannten Fetten, Tranen und Ölen durch Umesterung erhalten werden.

Fettsäuremethylestergemische im Sinne der vorliegenden Erfindung bestehen aus Fettsäuremethylestern, die geradkettig und/oder verzweigtkettig, gesättigt und/oder ungesättigt sind und einen Kohlenstoffekttenbereich von 8 bis 22 Kohlenstoffatomen aufweisen.

Gemäß dem erfindungsgemäßen Verfahren wird die katalytische Hydrierung der Fettsäuremethylestergemische in Gegenwart eines Katalysators durchgeführt, der - bezogen auf die oxidische Katalysatormasse - 30 bis 40 Gew.-% Kupfer, 23 bis 30 Gew.-% Chrom, 1 bis 10 Gew.-% Mangan, 1 bis 7 Gew.-% Barium und gegebenenfalls Züsalzlich 1 bis 5 Gew.% Zirkon und/oder Cer - jeweils bezogen auf oxidische Katalysatormasse - enthält. Die genannten Metalle liegen nach der Herstellung der Katalysatormassen, die auf an sich aus dem Stand der Technik bekannte Art und Weise erfolgt, in Form ihrer Oxide vor. Die Oxidbildung erfolgt, wie aus dem Stand der Technik bekannt, im Verlauf des sogenannten "Calcinierens"`, d.h. durch thermische Zersetzung von Mineralsalzen der jeweiligen Metalle.

Im erfindungsgemäßen Verfahren setzt man zur Hydrierung der Fettsäuremethylestergemische einen Katalysator ein, der, bezogen auf die oxydische Katalysatormasse, 1 bis 10 Gew.-% SiO$_2$ enthält.

In einer bevorzugten Ausführungsform des erfindungsgemäßen Verfahrens hydriert man kontinuierlich Fettsäuremethylestergemische unter Verwendung eines Katalysators, der vorteilhafterweise 32 bis 38 Gew.-% Kupfer, bezogen auf die oxidische Katalysatormasse, enthält. Ebenso kann es bevorzugt sein, die Menge an Chrom in dem verwendeten Katalysator auf einen Bereich von 26 bis 29 Gew.-%, die Menge an Mangan auf einen Bereich von 1 bis 10 Gew.-%, die Menge an Barium auf einen Bereich von 1,5 bis 3 Gew.-% oder die Menge an Silicium auf einen Bereich von 1,5 bis 3 Gew.-%, jeweils bezogen auf die oxidische Katalysatormasse vor der Aktivierung, einzustellen. In einer besonders bevorzugten Ausführungsform wird zur katalytischen Hydrierung von Fettsäuremethylestern ein Katalysator eingesetzt, der- bezogen auf die oxidische Katalysatormasse - 32 bis 28 Gew.-% Kupfler, 26 bis 29 Gew.% chrom, 1 bis 6 Gew.% Mangan, 1,5 bis 3 Gew.% Barium und 1,5-3 Gew.% Silicium enthält. Mit derartigen Katalysatoren lassen sich erhebliche Aktivitätssteigerungen erzielen. Aus diesem Grunde ist die Verwendung eines derartigen Katalysators in dem erfindungsgemäßen Verfahren als besonders bevorzugt anzusehen.

In einer weiteren bevorzugten Ausführungsform des Verfahrens kann zur katalytischen Hydrierung von Fettsäuremethylestergemischen ein Katalysator verwendet werden, der- bezogen auf die oxidische Katalysatormasse- 32 bis 28 Gew.-% Kupfler, 26 bis 29 Gew.% chrom, 1 bis 6 Gew.% Mangan, 1,5 bis 3 Gew.% Barium bis 3 Gew.% Silicium und 2 bis 3 Gew.% Zirkon und/oder Cer enthält. Die Verwendung derartiger, zusätzlich dotierter Katalysatoren in dem Verfahren führt insbesondere bei der Hydrierung im Rieselbett zu einer erheblichen Erhöhung der Aktivität und Selektivität der Katalysatoren.

Erfindungsgemäße Katalysatoren können 1 bis 10 Gew.-% an Graphit zur verbesserten Verarbeitbarkeit der Granulat- und/oder Extrudatkörper enthalten. Vorzugsweise wird eine Menge von 5 Gew.-% Graphit vor der Granulierung dem calcinierten pulverförmigen Material zugesetzt und mit diesem innig vermischt.

Erfindungsgemäß wurde eine Verbesserung des Verfahrens dadurch erreicht, daß der Verwendete, die oben genannten Metalle in Form ihrer Oxide und Graphit enthaltende Katalysator unter Einsatz von 1 bis 10 Gew.-% eines oder mehrerer Binder in Granulat- oder Extrudatform gebracht wird. Bevorzugt wird dabei ein Einsatz von 10 Gew.-% eines oder mehrerer Binder. Als solche kommen für diesen Zweck aus dem Stand der Technik bekannte Verbindungen in Frage, wobei in dem erfindungsgemäß verwendeten Katalysator entweder ein oder auch mehrere Binder verwendet werden können. Besonders hat sich der Einsatz von Polyvinylacetat und/oder Methylmethacrylat als Binder bewährt. Im Gegensatz zu zahlreichen, aus dem Stand der Technik bekannten, schlecht riesel fähigen Katalysatormaterialien konnte für das erfindungsgemäße Verfahren ein Katalysator in Granulat- oder Extrudatform zur Verfügung gestellt werden, dessen aufgelockerte, poröse Struktur zur Erhöhung der Aktivität und Selektivität des Katalysators bei der Hydrierung der Fettsäuremethylestergemische insbesondere im Rieselbett in erheblichem Maße beiträgt. Bevorzugt wird als Binder für die Herstellung der Katalysatorgranulate oder Katalysatorextrudate Polyvinylacetat, wobei zur Katalysatorherstellung beispielsweise 40 Gew.-%ige Polyvinylacetat-Suspensionen verwendet werden, die im Handel erhältlich sind. Die calcinierten pulverförmigen Katalysatormaterialien werden nach guter Durchmischung mit derartigen Polyvinylacetat-Suspensionen in kleinen Mengen versetzt und bis zum Beginn des Aufbaus von Agglomeratkörnern gemischt. 1m Anschluß daran wird das Agglomerate enthaltende Pulver beispielsweise in einem Lochwalzengranulator zu kleinen Granulaten verdichtet, wobei dieser Prozeß an sich aus dem Stand der Technik bekannt ist. Auf ebenfalls an sich bekannte Weise werden die Granluate getrocknet, wobei sich Restfeuchten von 10 bis 15 % einstellen lassen. Die aus diesem Vorgang resultierenden Granulate werden gesiebt, wobei für das erfindungsgemäße Verfahren Kornfraktionen bestimmter Korngröße ausgesiebt werden. Bei der Anwendung des erfindungsgemäßen Verfahrens zur katalytischen Hydrierung von Fettsäuremethylestergemischen in der Rieselfahrweise werden mit Vorteil Katalysator-Kornfraktionen eingesetzt, deren Korngröße im Bereich von 0,6 bis 3,0 mm liegt.

Die Katalysatoren können auch in Tablettenform, beispielsweise der Größe 4 x 4 mm, gepreßt werden. Zur Härtung dieser Tabletten werden diese 6 h bei einer Temperatur von 200 bis 280°C an der Luft getempert. Die nach der BET-Methode (Z. Anal. Chem. 288 (1968), 187-193) bestimmte spezifische Oberfläche betrug $40 \pm 10$ m$^2$/g.

Die in dem erfindungsgemäßen Verfahren zur Hydrierung von Fettsäuremethylestergemischen verwendbaren granulierten Katalysatoren weisen eine spezifische Oberfläche im Bereich von 30 bis 50 m$^2$/g auf. Die beschriebene Art der Vorgranulierung führt zu einer speziellen, aufgelockerten Porenstruktur, die sich in einer Erhöhung des Porennutzungsgrades auswirkt.

Im Verlaufe der Untersuchungen zu dem erfindungsgemäßen Verfahren zur Hydrierung von Fettsäuremethylestergemischen hat es sich gezeigt, daß es besonders bevorzugt ist, die Fettsäuremethylestergemische in Gegenwart eines Katalysators mit Wasserstoff umzusetzen, dessen Granulat-, Extrudat- oder Tablettenkörper einen Durchmesser von 1 bis 6 mm und eine Länge von 1 bis 6 mm aufweisen. Derartige Granulat- bzw. Extrudatkörper (Tabletten) zeigen eine ausgezeichnete Aktivität und Selektivität bei der Umsetzung der Fettsäuremethylestergemische mit Wasserstoff zu langkettigen Fettalkoholen und lassen sich zudem ohne Probleme von den Reaktionsprodukten abtrennen. Außerdem sind die mit diesen Katalysatoren erzielbaren Standzeiten deutlich besser als die Standzeiten der aus dem Stand der Technik bekannten Katalysatoren, die zudem den Nachteil aufwiesen, daß sie zum Teil bei der Reaktion zerfielen und dadurch nur unter großen Problemen von den Reaktionsprodukten abtrennbar waren.

Ein weiterer Faktor, der die Aktivität bzw. Selektivität der erfindungsgemäß eingesetzten Katalysatoren stark beeinflußt, ist das Porenvolumen der Katalysator-Formkörper. Es zeigte sich, daß das Porenvolumen der erfindungsgemäß verwendbaren Katalysatoren in einem Optimalbereich liegen muß, um in dem erfindungsgemäßen Verfahren zur Hydrierung von Fettsäuremethylestergemischen optimale Ergebnisse zu erbringen. In einer bevorzugten Ausführungsform des erfindungsgemäßen Verfahrens werden metallhaltige Katalysatoren verwendet, deren Porenvolumen im Bereich von 0,4 bis 0,6 cm$^3$/g liegt. Vorteilhafterweise

trägt ein Porenvolumen in diesem Bereich ebenfalls zu einer Erhöhung der Aktivität und Selektivität der Hydrierkatalysatoren bei: Sowohl im Rieselbettreaktor als auch im Sumpfphasenreaktor lassen sich hohe Aktivitäten und Selektivitäten erzielen; gleichzeitig wiesen derartige Katalysatoren in dem erfindungsgemäßen Verfahren eine ausgesprochen hohe Standzeit auf und führten nicht zu Problemen bei der Trennung von Katalysator und Reaktionsprodukten.

Die in dem erfindungsgemäßen Verfahren verwendeten Katalysatoren werden üblicherweise vor ihrem Einsatz in der Hydrierung von Fettsäuremethylestergemischen mit Wasserstoff oder einem Wasserstoff enthaltenden Gasgemisch aktiviert. Aus ökonomischen Gründen wird mit Vorteil zur Aktivierung der Katalysatormassen ein Gasgemisch verwendet, das überwiegend aus einer Stickstoff-/Wasserstoff-Gasmischung besteht. Vorteilhafterweise kann eine derartige Aktivierung - wie aus dem Stand der Technik bekannt - so durchgeführt werden, daß die Katalysatormassen nach der Herstellung im Stickstoffstrom bei erhöhter Temperatur getrocknet werden und dem trocknenden Gas in steigenden Mengen Wasserstoff zur Aktivierung beigemischt wird. Dabei kann der Wasserstoffanteil in dem aktivierenden Gasgemisch im Bereich von 0,1 bis 10 Vol.-% liegen. Die Aktivierung der Katalysatoren kann dabei sowohl in situ als auch in von dem Reaktionsgefäß getrennten Gefäßen durchgeführt werden.

Die Reaktionstemperaturen der Hydrierung von Fettsäuremethylestergemischen gemäß der vorliegenden Erfindung liegen im Bereich von 160 bis 270°C, bevorzugt im Bereich von 180 bis 240°C. Bei der Temperaturführung der Reaktion ist allgemein zu beachten, daß die Hydrierung der Fettsäuremethylestergemische zu entsprechenden Fettalkoholen eine exotherme chemische Reaktion ist. Bei der Temperaturführung muß also darauf geachtet werden, daß die entstehende Reaktionswärme in üblicher Weise abzuführen ist.

Das erfindungsgemäße Verfahren zur Hydrierung von Fettsäuremethylestergemischen ist außerdem dadurch gekennzeichnet, daß man ein Molverhältnis von Wasserstoff zu Fettsäurerest im Fettsäuremethylester-Substrat von 10 : 1 bis 500 : 1 einstellt. Dies bedeutet, daß die Durchsatzmenge an Wasserstoffgas, gemessen in Mol/Stunde, 10- bis 500mal höher liegt als die Durchsatzmenge an Fettsäuremethylester, gemessen in Mol Fettsäurerest/Stunde.

Die Vorteile des erfindungsgemäßen Verfahrens sind darin zu sehen, daß durch eine Optimierung sowohl der chemischen Zusammensetzung als auch des physikalischen Aufbaus der verwendeten Katalysatoren auf Kupferchromit-Basis das Verfahren zur Herstellung von Fettalkoholen vereinfacht wird, da die destillative Auftrennung der Ausgangsgemische der Fettsäuremethylester entfallen kann.

Der Einsatz des erfindungsgemäßen Katalysators in Verbindung mit der beschriebenen Verfahrensweise erlaubt die Hestellung von Fettalkoholen nach Umesterung ohne vorherige Auftrennung in die einzelnen Siedeschnitte.

Es werden dadurch Investitionen für Tankzwischenlager und zusätzliche Fraktionierkolonnen eingespart, sowie aufgrund der höheren Selektivität des Katalysators im Vergleich zum bisher verwendeten eine höhere Wertschöpfung erzielt.

Die Erfindung wird durch die nachfolgenden Beispiele näher erläutert.

Ausführungsbeispiel

Herstellung eines Katalysators:

Bei Temperaturen von 30 bis 90°C wurden 84,93 g Ba(NO$_3$)$_2$, 3493 g Cu(NO$_3$)$_2$ . 2 H$_2$O, 294,43 g Mn-(NO$_3$)$_2$ . 4 H$_2$O und 62,3 g SiO$_2$ in Form eines 40 Gew.%igen Kieselsäuresols in 9 l entsalztem Wasser unter starkem Rühren gelöst. In einem zweiten Behälter wurden 1639 g CrO$_3$ in 9 l entsalztem Wasser unter den gleichen Bedingungen gelöst und anschließend 3650 g einer 25 %igen Ammoniaklösung hinzugegeben. Danach wurde die Barium, Mangan und Kupfer enthaltende Lösung bei Temperaturen von 30 bis 90°C in die vorgelegte Ammoniumchromatlösung gepumpt, wobei aus der Lösung ein Gemenge von Bariumchromat, Manganhydroxid, Siliciumhydroxid und Kupferchromat ausfiel. Die Fällung war beendet bei Absinken des pH-Wertes unter 7.

Das Präzipitat wurde in einer Rahmenfilterpresse abfiltriert und mit entsalztem Wasser nitratfrei gewaschen. Der Filterkuchen wurde über Nacht bei 90 bis 120°C getrocknet und danach in einer Schneidmühle zu grobem Pulver gemahlen. Das resultierende Chromatpulver wurde im Drehrohrofen bei Temperaturen von 300 bis 500°C thermisch zum Chromit zersetzt ("calciniert"). Das calcinierte, pulverförmige Material hatte folgende chemische Zusammensetzung:

Cu: 38 ± 0,5 %;
Cr: 29 ± 0,5 %;
Mn: 2,5 ± 0,5 %;

Ba: 1,9 ± 0,5 % und
Si: 1 ± 0,3 %.

Einem Liter des Pulvers wurden 5 Gew.-% Graphit zugesetzt und im Lödige-Mischer 15 min durchmischt. Anschließend wurden 10 Gew.-% einer 40 gew.-%igen Polyvinylacetatsuspension zugegeben und kurz bis zum Aufbaubeginn von Agglomeraten durchgemischt. Im Anschluß daran wurde das Pulver in einem Lochwalzengranulator zu kleinen Granulaten verdichtet, auf eine Restfeuchte von 10 bis 15 % getrocknet und zu einer 0,6- bis 2 mm-Kornfraktion gesiebt.

Das Granulat hatte ausgezeichnete Fließeigenschaften und konnte auf einer Rundläufer-Tablettenmaschine zu Tabletten von 3 bis 6 mm Durchmesser und 2 bis 4 mm Höhe verpreßt werden.

Nach der Härtung (6h, 200°C, an der Luft) resultierten Tabletten mit einer spezifischen Oberfläche (bestimmt nach BET) von 40 ± 10 $m^2/g$ und einem Porenvolumen von 0,4 bis 0,6 $cm^3/g$.

Beispiele 1 bis 3

0,5 l des gemäß dem Ausführungsbeispiel hergestellten und granulierten Katalysators mit einer mittleren Korngröße von 1 mm wurden in ein Reaktionsrohr mit dem Volumen von einem Liter und einem Innendurchmesser von 25 mm eingefüllt und zur gleichmäßigen Verteilung der flüssigen Phase mit 2 mm Glaskugeln überschichtet.

Nach Trocknung und Reduktion des Katalysators in einem Stickstoff/Wasserstoffstrom (Wasserstoffkonz. (max.) 1 %; Temperatur (max.) 200 °C) wurde bei 250 bar und 200 bis 220 °C kontinuierlich Wasserstoff im Gleichstrom mit einer Fettsäuremethylester-Mischung, die eine C-Kettenverteilung entsprechend der im Kokosöl enthaltenen Fettsäuren zwischen C8 bis C18 aufwies, von oben durch den Reaktor geleitet und das flüssige Reaktionsprodukt in einem zweistufigen Abscheidesystem aufgefangen. Die Reaktionsbedingungen und die Produktanalysen sind in Tabelle 1 zusammengefaßt:

## Versuchsergebnisse – Tabelle 1
## Kokosmethylester (C8 bis C18)-Hydrierung

Druck: 250 bar          Säurezahl        (SZ) = <1
Verseifungszahl (VZ): 249    Jodzahl          (JZ) =   8,2
                        Hydroxylzahl (OHZ) = 19,0

| Beispiele | 1 | 2 | 3 |
|---|---|---|---|
| Reaktionstemperatur (°C) | 200 | 200 | 200 |
| LHSV* ($l \times l^{-1} \times h^{-1}$) | 2,0 | 3,0 | 4,0 |
| $H_2$: Substrat ($mol \times mol^{-1}$) | 100 | 75 | 50 |
| Produkt-Verseifungszahl | 0,3 | 0,8 | 12 |
| **Produktzusammensetzung (Gew.-%)** | | | |
| Fettsäuremethylester | 0,1 | 0,3 | 4,7 |
| Fettalkohole | 85,9 | 85,7 | 81,9 |
| Kohlenwasserstoffe | 0,05 | 0,03 | 0,03 |
| Methanol | 13,9 | 13,9 | 13,3 |

LHSV* (liquid hourly space velocity)

6

Vergleichsbeispiele 1 bis 3

Die Kokosmethylester-Fraktionen im Siedebereich der C8 bis C10 und der C12 bis C14-Methylester bzw. der Talgfettsäuremethylester (C16 bis C18) wurden unter Reaktionsbedingungen wie in Tabelle 2 beschrieben, hydriert. Die Versuche wurden in der gleichen Anlage und mit dem gleichen Katalysator des Beispiels 1 durchgeführt.

## Versuchsergebnisse - Tabelle 2
## Kokosmethylester-Hydrierung
### Druck: 250 bar

| Vergleichsbeispiele | 1 | 2 | 3 |
|---|---|---|---|
| Substrat (Fraktion) | C8-C10 | C12-C18 | C16-C18 |
| Reaktionstemperatur °C | 200 | 200 | 200 |
| LHSV* $(l \times l^{-1} \times h^{-1})$ | 4,0 | 2,0 | 4,0 |
| $H_2$: Substrat $(mol \times mol^{-1})$ | 50 | 100 | 50 |
| Produkt-Verseifungszahlen | 35 | 0,4 | 0,8 |
| Produktzusammensetzung (Gew.-%) | | | |
| Fettsäuremethylester | 11,7 | 0,2 | 0,4 |
| Fettalkohole | 70,6 | 86,3 | 87,6 |
| Kohlenwasserstoffe | 0,02 | 0,05 | 0,03 |
| Methanol | 17,6 | 13,4 | 11,95 |

Wie aus dem Vergleichsbeispiel 1 ersichtlich wird, lassen sich Methylester kurzkettiger Fettsäuren weniger gut hydrieren als längerkettige (Vergleichsbasis: Volumen Substrat/Volumen Katalysator/Stunde). Die Beispiele 1 bis 3 zeigen, daß die Hydrierung von unfraktioniertem Kokosmethylester trotz unterschiedlicher Reaktivität der einzelnen Methylester zu keiner Selektivitätsverschlechterung bezüglich Kohlenwasserstoff-Bildung führt; die Kohlenwasserstoff-Anteile liegen bei den angegebenen Reaktionsbedingungen deutlich unter 0,1 Gew.-%, so daß eine Abtrennung durch Destillation nicht erforderlich wird.

**Patentansprüche**

1. Verfahren zur katalytischen Hydrierung von Fettsäuremethylestergemischen mit kurzkettigen und langkettigen Fettsäureresten im Kohlenstoffbereich von 8 bis 22 Kohlenstoffatomen bei erhöhten Reaktionstemperaturen unter Verwendung stückiger und/oder gekörnter, Kupferchromit als Hauptbestandteil enthaltender Katalysatoren, **dadurch gekennzeichnet,** daß man diese ohne vorherige Auftrennung in einzelne Siedeschnitte zusammen mit Wasserstoff bei Drucken von 100 bis 300 bar und Temperaturen von 160 bis 270°C bei Molverhältnissen von Wasserstoff : Fettsäureresten im Fettsäuremethylester-Einsatz von 10 : 1 bis 500 : 1 kontinuierlich über Katalysatoren umsetzt, die
30 bis 40 Gew.-% Kupfer,
23 bis 30 Gew.-% Chrom,
1 bis 10 Gew.-% Mangan

1 bis 10 Gew.-% Silicium und
1 bis 7 Gew.-% Barium
sowie gegebenenfalls zusätzlich
1 bis 5 Gew.-% Zirkon und/oder Cer
- jeweils bezogen auf oxidische Katalysatormasse - enthalten
und nach dem Calcinieren der die Katalysatormasse bildenden Komponenten mit 1 bis 10 Gew.-% - bezogen auf oxidischen Katalysator - wenigstens eines Binders und 1 bis 10 Gew.-% - bezogen auf oxidischen Katalysator - Graphit zu stückigen und/oder gekörnten Formlingen, die eine spezifische Oberfläche im Bereich von 30 bis 50 m$^2$/g, ein Porenvolumen im Bereich von 0,4 bis 0,6 cm$^3$/g, und bei stückigen Formlingen einen Durchmesser von 1 bis 6 mm und eine Länge von 1 bis 6 mm, bei gekörnten Formlingen eine Korngröße im Bereich von 0,6 bis 3,0 mm aufweisen, umgewandelt und mit Wasserstoff oder einem Wasserstoff enthaltenden Gasgemisch aktiviert worden sind.

2. Verfahren nach Anspruch 1, **dadurch gekennzeichnet,** daß man Fettsäuremethylestergemische einsetzt.

3. Verfahren nach den Ansprüchen 1 und 2, **dadurch gekennzeichnet,** daß man Fettsäuremethylestergemische unter Verwendung eines 32 bis 38 Gew.-% - bezogen auf die oxidische Katalysatormasse - Kupfer enthaltenden Katalysators einsetzt.

4. Verfahren nach den Ansprüchen 1 und 2, **dadurch gekennzeichnet,** daß man Fettsäuremethylestergemische unter Verwendung eines 26 bis 29 Gew.-% - bezogen auf die oxidische Katalysatormasse - Chrom enthaltenden Katalysators einsetzt.

5. Verfahren nach den Ansprüchen 1 und 2, **dadurch gekennzeichnet,** daß man Fettsäuremethylestergemische unter Verwendung eines 1,5 bis 3 Gew.-% - bezogen auf die oxidische Katalysatormasse - Barium enthaltenden Katalysators einsetzt.

6. Verfahren nach den Ansprüchen 1 und 2, **dadurch gekennzeichnet,** daß man Fettsäuremethylestergemische unter Verwendung eines 1,5 bis 3 Gew.-% - bezogen auf die oxidische Katalysatormasse - Silicium enthaltenden Katalysators einsetzt.

7. Verfahren nach den Ansprüchen 1 und 2, **dadurch gekennzeichnet,** daß man Fettsäuremethylestergemische unter Verwendung eines Katalysators einsetzt, der - bezogen auf die oxidische Katalysatormasse -
32 bis 28 Gew.-% Kupfer,
26 bis 29 Gew.-% Chrom,
1 bis 6 Gew.-% Mangan,
1,5 bis 3 Gew.-% Barium und
1,5 bis 3 Gew.-% Silicium
enthält.

8. Verfahren nach den Ansprüchen 1 bis 7, **dadurch gekennzeichnet,** daß man Fettsäuremethylestergemische unter Verwendung eines Katalysators einsetzt, der - bezogen auf die oxidische Katalysatormasse -
32 bis 28 Gew.-% Kupfer,
26 bis 29 Gew.-% Chrom,
1 bis 6 Gew.-% Mangan,
1,5 bis 3 Gew.-% Barium,
1,5 bis 3 Gew.-% Silicium und
2 bis 3 Gew.-% Zirkon und/oder Cer
enthält.

9. Verfahren nach den Ansprüchen 1 bis 8, **dadurch gekennzeichnet,** daß man als Binder Polyvinylacetat und/oder Methylmethacrylat einsetzt.

10. Verfahren nach den Ansprüchen 1 bis 9, **dadurch gekennzeichnet,** daß man den Katalysator mit

EP 0 300 347 B1

einem 0,1 bis 10 Vol.-% Wasserstoff enthaltenden $N_2$-/$H_2$-Gasgemisch aktiviert.

11. Verfahren nach den Ansprüchen 1 bis 10, **dadurch gekennzeichnet,** daß man die Hydrierung bei 180 bis 240°C durchführt.

**Claims**

1. A process for the catalytic hydrogenation of fatty acid methyl ester mixtures containing short-chain and long-chain fatty acid residues in the $C_8$ to $C_{22}$ carbon chain range at elevated reaction temperatures using pelleted and/or granular catalysts containing copper chromite as principal constituent, characterized in that, without being separated up beforehand into individual boiling cuts, the mixtures are continuously reacted with hydrogen under pressures of 100 to 300 bar and at temperatures of from 160 to 270°C with molar ratios of hydrogen to fatty acid residues in the fatty acid methyl ester substrate of from 10:1 to 500:1, the reaction being carried out over catalysts which contain

   30 to 40% by weight copper,
   23 to 30% by weight chromium,
   1 to 10% by weight manganese,
   1 to 10% by weight silicon and
   1 to 7% by weight barium
   and, optionally,
   1 to 5% by weight zirconium and/or cerium
   (% by weight, based in each case on oxidic catalyst mass)
   and which, after calcination of the components forming the catalyst mass, have been converted into pellets and/or granules having a specific surface of 30 to 50 $m^2$/g, a pore volume of 0.4 to 0.6 $cm^3$/g and, in the case of pellets, a diameter of 1 to 6 mm and a length of 1 to 6 mm, in the case of granules a grain size of 0.6 to 3.0 mm, with 1 to 10% by weight, based on oxidic catalyst, of at least one binder and 1 to 10% by weight, based on oxidic catalyst, graphite and activated with hydrogen or a hydrogencontaining gas mixture.

2. A process as claimed in claim 1, characterized in that fatty acid methyl ester mixtures are used.

3. A process as claimed in claims 1 and 2, characterized in that fatty acid methyl ester mixtures are hydrogenated using a catalyst containing 32 to 38% by weight, based on oxidic catalyst mass, of copper.

4. A process as claimed in claims 1 and 2, characterized in that fatty acid methyl ester mixtures are hydrogenated using a catalyst containing 26 to 29% by weight, based on oxidic catalyst mass, of chromium.

5. A process as claimed in claims 1 and 2, characterized in that fatty acid methyl ester mixtures are hydrogenated using a catalyst containing 1.5 to 3% by weight, based on oxidic catalyst mass, of barium.

6. A process as claimed in claims 1 and 2, characterized in that fatty acid methyl ester mixtures are hydrogenated using a catalyst containing 1.5 to 3% by weight, based on oxidic catalyst mass, of silicon.

7. A process as claimed in claims 1 and 2, characterized in that fatty acid methyl ester mixtures are hydrogenated using a catalyst containing
   32 to 28% by weight copper,
   26 to 29% by weight chromium,
   1 to 6% by weight manganese,
   1.5 to 3% by weight barium and
   1.5 to 3% by weight silicon,
   based on oxidic catalyst mass.

8. A process as claimed in claims 1 to 7, characterized in that fatty acid methyl ester mixtures are hydrogenated using a catalyst containing

9

32 to 28% by weight copper,
26 to 29% by weight chromium,
1 to 6% by weight manganese,
1.5 to 3% by weight barium,
1.5 to 3% by weight silicon and
2 to 3% by weight zirconium and/or cerium,
based on oxidic catalyst mass.

9. A process as claimed in claims 1 to 8, characterized in that polyvinyl acetate and/or methyl methacrylate is/are used as binder.

10. A process as claimed in claims 1 to 9, characterized in that the catalyst is activated with an $N_2/H_2$ gas mixture containing 0.1 to 10% by volume hydrogen.

11. A process as claimed in claims 1 to 10, characterized in that the hydrogenation is carried out at 180 to 240°C.

**Revendications**

1. Procédé d'hydrogénation catalytique de mélanges d'esters méthyliques d'acides gras comportant des résidus ou restes d'acides gras à chaine courte et à chaine longue, dont le nombre d'atomes de carbone est compris dans l'intervalle de 8 à 22, à des températures de réaction élevées et en mettant en oeuvre des catalyseurs on morceaux et/ou en granulés, comprenant comme composant principal du chromite de cuivre, caractérisé en ce que l'on fait réagir continuellement ceux-ci avec l'hydrogène, sans séparation préalable, en phases de distillation individuelles, à des pressions comprises entre 100 et 300 bars et à des températures allant de 160 à 270 °C, l'hydrogène et les restes d'esters d'acides gras dans le ou les ester(s) méthylique(s) d'acides gras mis en oeuvre étant entre eux dans des rapports molaires compris entre 10:1 et 500:1, sous l'action de catalyseurs, qui renferment
30 à 40 % en poids de cuivre,
23 à 30 % en poids de chrome,
1 à 10 % en poids de manganèse,
1 à 10 % en poids de silicium et
1 à 7 % en poids de baryum,
ainsi que, le cas échéant, en plus
1 à 5 % en poids de zirconium et/ou de cérium
- dans chaque cas, par rapport à la masse de catalyseur oxydé,
et qui ont été transformés, après la calcination des composants formant la masse de catalyseur avec 1 à 10 % en poids (par rapport au catalyseur à l'état d'oxyde) d'au moins un liant et avec 1 à 10 % en poids (par rapport au catalyseur à l'état d'oxyde) de graphite, en morceaux et/ou en granulés, qui présentent une surface spécifique comprise dans l'intervalle de 30 à 50 $m^2/g$, un volume de pore se situant dans la plage de 0,4 à 0,6 $cm^3/g$, et pour les agglomérés en morceaux, un diamètre de 1 à 6 mm et une longueur se situant de 1 à 6 mm, et pour les agglomérés en granulés, une grosseur de granulé de 0,6 à 3,0 mm, et qui ont été activés avec de l'hydrogène ou avec un mélange gazeux renfermant de l'hydrogène.

2. Procédé selon la revendication 1, caractérisé en ce que l'on met en oeuvre des mélanges d'esters méthyliques d'acides gras.

3. Procédé selon les revendications 1 et 2, caractérisé en ce que l'on met en oeuvre des mélanges d'esters méthyliques d'acides gras, en utilisant 32 à 38 % en poids, par rapport à la masse de catalyseur à l'état d'oxyde, d'un catalyseur renfermant du cuivre.

4. Procédé selon les revendications 1 et 2, caractérisé en ce que l'on mot en oeuvre des mélanges d'esters méthyliques d'acides gras, en utilisant 26 à 29 % en poids, par rapport à la masse de catalyseur à l'état d'oxyde, d'un catalyseur renfermant du chrome.

5. Procédé selon les revendications 1 et 2, caractérisé en ce que l'on met en oeuvre des mélanges d'esters méthyliques d'acides gras, en utilisant 1,5 à 3 % en poids, par rapport à la masse de catalyseur à l'état d'oxyde, d'un catalyseur renferment du baryum.

6. Procédé salon les revendications 1 et 2, caractérisé en ce que l'on met en oeuvre des mélanges d'esters méthyliques d'acides gras, en utilisent 1,5 à 3 % en poids, par rapport à la masse de catalyseur à l'état d'oxyde, d'un catalyseur renfermant du silicium.

7. Procédé selon les revendications 1 et 2, caractérisé en ce que l'on met en oeuvre des mélanges d'esters méthyliques d'acides gras, en utilisant un catalyseur, qui renferme, par rapport à la masse de catalyseur à l'état d'oxyde,
32 à 28 % en poids de cuivre,
26 à 29 % on poids de chrome,
1 à 6 % en poids de manganèse,
1,5 à 3 % en poids de baryum et
1,5 à 3 % en poids de silicium.

8. Procédé selon les revendications 1 à 7, caractérisé en ce que l'on met en oeuvre des mélanges d'esters méthyliques d'acides gras, en utilisant un catalyseur, qui renferme, par rapport à la masse de catalyseur à l'état d'oxyde,
32 à 28 % en poids de cuivre,
26 à 29 % en poids de chrome,
1 à 6 % en poids de manganèse,
1,5 à 3 % en poids de baryum,
1,5 à 3 % en poids de silicium et
2 à 3 % en poids de zirconium et/ou de cérium.

9. Procédé selon les revendications 1 à 8, caractérisé en ce que l'on utilise comme liant, de l'acétate de polyvinyle et/ou du méthacrylate de méthyle.

10. Procédé selon les revendications 1 à 9, caractérisé en ce que l'on active le catalyseur ou moyen d'un mélange gazeux de $N_2$ et de $H_2$ renfermant 0,1 à 10 % en vol. d'hydrogène.

11. Procédé selon les revendications 1 à 10, caractérisé en ce que l'on réalise l'hydrogénation à une température de 180 à 240 °C.